# EUROPÄISCHE PATENTSCHRIFT

(11) **EP 1 069 914 B1**
(45) Veröffentlichungstag und Bekanntmachung des Hinweises auf die Patenterteilung: **13.06.2007**
(21) Anmeldenummer: 99919217.2
(22) Anmeldetag: 08.04.1999
(51) Int. Cl.: A61K 47/36, A61K 9/20, A61K 31/70, A61K 31/52, A61K 31/40

(54) **RETARDTABLETTE HERGESTELLT AUS LINEAREN WASSERUNLÖSLICHEN POLYSACCHARIDEN**
CONTROLLED RELEASE TABLET PRODUCED FROM LINEAR WATER-INSOLUBLE POLYSACCHARIDES
COMPRIME A LIBERATION CONTROLEE PRODUIT A PARTIR DE POLYSACCHARIDES LINEAIRES INSOLUBLES DANS L'EAU

(30) Priorität: 09.04.1998 DE 19816070
(43) Veröffentlichungstag der Anmeldung: 24.01.2001
(73) Patentinhaber: Südzucker AG Mannheim/Ochsenfurt, 68165 Mannheim (DE)
(72) Erfinder: BENGS, Holger, D-60598 Frankfurt am Main (DE); SCHUTH, Silke, D-56412 Ruppach-Goldhausen (DE); GRANDE, Jürgen, D-65812 Bad Soden (DE); BÖHM, Gitte, D-60439 Frankfurt am Main (DE); SCHNELLER, Arnold, D-64409 Messel (DE)
(74) Vertreter: Luderschmidt, Schüler & Partner
(86) Internationale Anmeldenummer: PCT/EP1999/002386
(87) Internationale Veröffentlichungsnummer: WO 1999/052558

(56) Entgegenhaltungen:
- WO-A-94/06416
- WO-A-96/41617
- WO-A-99/11695
- MASUMOTO K. ET AL.: "Sustained-Release Dosage Forms Containing Chlorpheniramine Maleate with Water-Insoluble Glucan" CHEM. PHARM. BULL., Bd. 32, Nr. 3, 1984, Seiten 1055-1062, XP002112248
- MASUMOTO K. ET AL.: "In vitro Dissolution Profile and in Vivo Absorption Study of Sustained-Release Tablets Containing Chlorpheniramine Maleate with Water-Insoluble Glucan" CHEM. PHARM. BULL., Bd. 32, Nr. 9, 1984, Seiten 3720-3723, XP002112249
- MASUMOTO K. ET AL.: "Directly Compressed Tablets containing Water-insoluble Glucan and Microcrystalline Cellulose in Addition to Lactose" CHEM. PHARM. BULL., Bd. 31, Nr. 1, 1983, Seiten 209-213, XP002112250

## Beschreibung

Die vorliegende Erfindung betrifft Retardtabletten aus linearen wasserunlöslichen Polysacchariden sowie deren Verwendung, insbesondere zur kontrollierten Abgabe von Wirkstoffen.

In der modernen pharmazeutischen Technologie sind Formulierungen von Wirkstoffträgern (Excipient) von Bedeutung, deren Anwendungsform gezielt Einfluß auf die Bioverteilung, Bioverfügbarkeit, Bioverträglichkeit und Resorption nehmen. Zudem müssen Wirkstoffträger gute mechanische Eigenschaften aufweisen, wie ausreichende Härte und Resistenz gegenüber Zug und Spannung.
Obwohl einige Verbindungen bereits selbst zu kompakten stabilen Massen gepreßt werden können (z. B. Saccharose oder Lactose) sind Ingredentien - auch Tablettenhilfsstoffe - erforderlich, wie Binde-, Füll-, Gleit- und Zusatzmittel. Typische Trockenbinder zur Stabilitätssteigerung, die hier Anwendung finden, sind:
Calciumphosphate, mikrokristalline Cellulose (z, B. Avicel^{®}, PH 102^{®}, speziell Celsphere), Polyvinylpyrrolidone (z. B. Kollidon^{®}, Luviskol VA 64^{®}, Plasdone^{®}),
Mais, Weizen- oder Kartoffelstärke, derivatisierte Polysaccharide, sogenannte Gums, (z. B. Xanthan Gum), Cellulosederivate (z. B. Hydroxypropylmethylcellulose: Klucel^{®}) oder Ethylcellulose (Aqualon^{®}).
Zudem müssen die Wirkstoffträger im Kontakt mit Körperflüssigkeiten in optimaler und kontrollierter Weise im Organismus zerfallen. Daher werden zur Zerfallssteuerung oftmals sogenannte Desintegrantien hinzugefügt. Typische Verbindungen für diesen Zweck sind Maisstärke, gelatinisierte Stärke und
Stärkemodifikationen. Ebenfalls einsetzbar sind Substanzen, die durch Wasseraufnahme und einhergehender Quellung eine Sprengkraft entwickeln.
Hierunter fallen vernetzte Polyvinylpyrrolidone (Kollidon CL^{®}), Carboxymethylcellulose und deren Calciumsalze oder Galactomannane.

Mit manchen Verbindungen (z. B. Avicel^{®} und PH 102^{®}) gelingt es sowohl die notwendige mechanische Stabilität zu erlangen als auch den Tablettenzerfall zu steuern.

Spezielle Stärken, einschließlich Amylose, werden als vorteilhafter Wirkstoffträger zur Tablettenformulierung beschrieben (Journal of Pharmaceutical Sciences 55 (1966), 340). Jedoch erweist sich die verwendete Nepol-Amylose (A. E. Stanley Manufacturing Co., USA) als nachteilig, da die Wirkstoffe nicht erschöpfend freigesetzt werden und der Wirkstoffträger einen hohen Wassergehalt (10 - 12 %) aufweist, weshalb hydrolytisch labile Wirkstoffe nicht formuliert werden können. Insbesondere vernetzte Amylose (Vernetzungsgrad 15 %) wird als überlegenes Bindemittel beschrieben (S.T.P. Pharma Sciences 4 (1994), 329-335 und Journal of Controlled Release 15, (1991) 39-46, Journal of Controlled Release 15, (1991)39-46), die aufgrund ihrer Wasseraufnahmekapazität als Zerfallsbeschleuniger wirkt. In WO 94/21236 wird vernetzte Amylose (Vernetzungsgrad 25 %) als Binde- und Desintegrationsmittel verwendet. Ein hoher Vernetzungsgrad wirkt sich jedoch nachteilig auf die biologische Verträglichkeit aus. Als Vernetzungsmittel wird bis zu 30 Gewichtsprozenten das unverträgliche Epichlorhydrin verwendet. Bereits geringe Vernetzungen im Bereich von wenigen Prozenten führen zu einer schnell anwachsenden Reaktionsträgheit, so daß mit verbleibenden Resten an nicht abreagiertem Vernetzer gerechnet werden muß.
Alle bisher auf dem Markt befindlichen stärke- und amylosehaltigen Wirkstoffträger verwenden pflanzliche Ursprungsquellen.
Hierbei ist von Nachteil, daß diese Biopolymere wie alle natürlich vorkommenden Substanzen erhebliche Schwankungen im Aufbau und Struktur aufweisen und daher die erforderliche Reproduzierbarkeit und damit gleichbleibende Produktqualität nicht gewährleistet ist, auch im Hinblick auf eine kontrollierte Wirkstoffabgabe.
Im Falle nativer Stärke schwankt je nach Herkunft der Gehalt an Amylose und Amylopektin beträchtlich. Zum Beispiel enthält Stärke aus Kartoffeln ca. 20 Gew. % Amylose und ca. 80 Gew. % Amylopektin während Stärke aus Mais ca. 50 Gew. % Amylose und ca. 50 Gew % Amylopektin aufweist. Zusätzliche Varianz innerhalb einer Pflanzengemeinschaft entstehen durch Bodenbeschaffenheit, Düngeraufnahme, saisonal klimatische Unterschiede etc.
Zudem können Amylose, ein 1,4 verknüpftes Polyglukan, mit einem Molekulargewicht von etwa 50.000 bis 150.000 Dalton, und Amylopektin, ein hochverzweigtes 1,4- und 1,6-verknüpftes Polyglukan, mit einem Molekulargewicht von etwa 300.000 bis 2.000.000 Dalton, breite Molekulargewichtsverteilungen aufweisen.
Die Übergänge von hochverzweigt zu linear sind fließend und variieren im pflanzlichen Ursprungsmaterial, so daß eine scharfe Abgrenzung nahezu unmöglich ist. Insbesondere Wirkstoffträger, die noch Amylopektin enthalten bewirken aufgrund der Verzweigungen unregelmäßige Quellungen, wodurch die Trägerstabilität beeinträchtigt wird. Amylopektin wird daher zumeist mittels enzymatischer Entzweigung (Journal of Controlled Release 45, (1997) 25-33 und EP 0499 648 B1 = US 5,468,286) aufwendig entfernt.

Neben diesen ausgeprägten Nachteilen, der breiten Molekulargewichtsverteilung oder Mischungen aus Polymeren unterschiedlicher räumlicher Anordnung, enthalten native Polymere weitere Bestandteile wie niedermolekulare Verbindungen, z. B. Fette und Öle, die nur schwer abtrennbar sind und sich in der weiteren Verarbeitung und Anwendung nachteilig auswirken (z. B. US 3,490,742). Insbesondere müssen ausbeutemindernde Arbeitsschritte durchgeführt werden, wobei zum Teil Verunreinigungen nicht vollständig eliminiert werden können.

Bekannt sind ebenfalls Versuche Biopolymere, so auch Stärke, zu optimieren, indem die Herkunftspflanze gentechnisch verändert wird. WO 94/03049 beschreibt die Herstellung und Verwendung hochamylosehaltiger Stärke aus gentechnisch verändertem Mais. Dessen ungeachtet bleiben die Nachteile der Uneinheitlichkeit und Verunreinigung.

Die Reproduzierbarkeit und Qualität ist maßgeblich von der Einheitlichkeit und Reinheit abhängig. Zur Gewährleistung von Produkten hoher Qualität müssen diese Ausgangsstoffe klar definierbar und charakterisierbar sein.

Die Veröffentlichungen K. Masumoto et al. Sustained-Release Dosage Forms Containing Chlorpheniramine Maleate with Water-Insoluble Glucan Chem. Pharm, Bull. 1984, 32(3), 1055-1062, K. Masumoto et al. In Vitro Profile and in Vivo Absorption Study of Sustained-Release Tablets Containing Chlorpheniramine Maleate with Water-Insoluble Glucan Chem. Pharm. Bull. 1984, 32(9), 3720-3723 und K. Masumoto et al. Directly Compressed Tablets containing Water-insoluble Glucan and Microcrystalline Cellulose in Addition to Lactose Chem. Pharm, Bull. 1983, 31(1), 209-213 beschäftigen sich mit der Eignung eines wasserunlöslichen, stark verzweigten Glukans als Retardmittel, das durch den Organismus *Streptococcus mutans* hergestellt werden kann.

WO 94/01091 beschreibt Retardtabletten aus wasserlöslichen Materialien, bei denen ohne Zusatz von weiteren Zusatzstoffen eine Wirkstoffabgabe nicht möglich ist.

WO 96/41617 offenbart Retardtabletten, die aus zwei Fraktionen bestehen können, wobei die erste Fraktion den Wirkstoff in gut wasserlöslicher Form enthält, während die zweite Fraktion den gleichen Wirkstoff in weniger gut löslicher Form umfasst.

Die vorliegende Erfindung hat die Aufgabe unter Vermeidung obiger Nachteile ein Retardmaterial bereitzustellen, welches als Retardtablette in einer pharmazeutischen Zusammensetzung zur kontrollierten Abgabe von Wirkstoffen verwendet werden kann, vorzugsweise zur oralen Applikation.

Die Aufgabe wird dadurch gelöst, daß als Retardmaterial wasserunlösliche lineares Polysaccharide verwendet werden, welche biokompatible, chemisch inerte, druckstabile Ausgangsprodukte darstellen, die die kontrollierte Wirkstoffabgabe ohne weitere Zusatzstoffe ermöglichen.

Dabei wird als Ausgangsprodukt lineares wasserunlösliches Poly(1,4-alpha-D-glukan) verwendet, das ganz oder teilweise in Form von vorzugsweise sphärischen Mikropartikeln vorliegt,

"Retardtablette" im Sinne der vorliegenden Erfindung sind insbesondere Tabletten, Dragees, Pillen, Pellets, Preßlinge, Plättchen, diskotische Scheiben u. ä,, deren Formulierung der Kompression bedarf. Ebenso einzubeziehen sind Kapseln, welche mit dem Retardmaterial gefüllt werden.

Als Retardmaterial wird im folgenden lineares wasserunlösliches angesehen.

Poly(1,4-alpha-D-glukan), angesehen.

Diese sind stets in der gleichen Art miteinander verknüpft. Jede so definierte Grundeinheit hat genau zwei Verknüpfungen, jeweils eine zu einem anderen Monomer. Davon sind ausgenommen die beiden Grundeinheiten, die den Anfang und das Ende des Polysaccharids bilden. Diese Grundeinheiten haben nur eine Verknüpfung zu einem weiteren Monomer. Bei drei oder mehr Verknüpfungen (kovalente Bindungen) eines Monomers zu einer anderen Gruppe, bevorzugt einer weiteren Saccharideinheit, spricht man von einer Verzweigung. Von jedem Saccharidbaustein im Polymerrückgrat gehen dann mindestens drei glykosidische Bindungen ab.
Erfindungsgemäß treten Verzweigungen nicht oder nur in so untergeordnetem Maß auf, daß sie bei den vorliegenden sehr kleinen Verzweigungsanteilen im allgemeinen den herkömmlichen analytischen Methoden nicht mehr zugänglich sind. Dies ist zum Beispiel dann der Fall, wenn bezogen auf die Gesamtheit aller vorhandenen Hydroxygruppen auf einhundert Hydroxygruppen, die nicht zum Aufbau des linearen Polysaccharids benötigt werden, maximal fünf Hydroxygruppen durch Verknüpfungen zu anderen Saccharidbausteinen belegt sind.

Dabei ist der Verzweigungsgrad maximal (100 %), wenn an jeder Saccharideinheit die freien Hydroxygruppen (oder andere auftretende funktionelle Gruppen) weitere glykosidische (oder andere) Bindungen zu weiteren Sacchariden aufweisen. Der Verzweigungsgrad ist minimal (0 %), wenn an den Sacchariden außer den Hydroxygruppen, die die Linearität des Polymers bedingen, keine weiteren Hydroxygruppen durch chemische Reaktion verändert sind.

Besitzt die Grundeinheit drei oder mehr Verknüpfungen, wird von Verzweigung gesprochen. Dabei ergibt sich aus der Anzahl der Hydroxylgruppen pro 100 Grundeinheit, die nicht am Aufbau des linearen Polymerrückgrats beteiligt sind und die Verzweigungen ausbilden, der sogenannte Verzweigungsgrad.

Erfindungsgemäß weisen die linearen wasserunlöslichen Polysaccharide einen Verzweigungsgrad von weniger als 8 % auf, d.h. sie haben weniger als 8 Verzweigungen auf 100 Grundeinheiten, Vorzugsweise ist der Verzweigungsgrad kleiner 4 % und insbesondere maximal 1,5 %.

Der Verzweigungsgrad in 6-Position ist kleiner 4 %, vorzugsweise maximal 2 % und insbesondere maximal 0,5 % und der Verzweigungsgrad in den anderen nicht an der linearen Verknüpfung beteiligten Positionen, z.B. der 2- bzw. 3-Position ist vorzugsweise jeweils maximal 2 % und insbesondere maximal 1 %.
Besonders bevorzugt sind insbesondere Poly-alpha-D-Glucane die keine Verzweigungen aufweisen, bzw. deren Verzweigungsgrad so minimal ist, daß er mit herkömmlichen Methoden nicht mehr nachweisbar ist.

Erfindungsgemäß beziehen die Präfixe "alpha", "beta" oder "D" allein auf die Verknüpfungen, die das Polymerrückgrat ausbilden und nicht auf die Verzweigungen.

"Wasserunlöslichkeit" im Sinne der vorliegenden Erfindung bedeutet, daß keine erkennbare Löslichkeit der Verbindung unter Normalbedingungen (Raumtemperatur von 25 °C und ein Luftdruck von 101325 Pascal oder davon maximal 20 % abweichende Werte zugrunde liegt) besteht,

Im Fall des erfindungsgemäß verwendeten Poly(1,4-alpha-D-glukans) bedeutet dies, daß mindestens 98 % der eingesetzten Menge, bevorzugt eine Menge größer 99,5 %, in Wasser unlöslich ist. Dabei kann der Begriff Unlöslichkeit auch anhand folgender Beobachtung erläutert werden. Erhitzt man 1 g des zu untersuchenden linearen Polysaccharids in 1 l entionisiertem Wasser auf 130 °C unter einem Druck von 1 bar, so bleibt die entstehende Lösung nur kurzzeitig, über wenige Minuten stabil. Beim Erkalten unter Normalbedingungen fällt die Substanz wieder aus. Nach weiterem Erkalten und Abtrennung mit der Zentrifuge unter Einkalkulation von experimentellen Verlusten, lassen sich auf diese Weise mindestens 66 % der eingesetzten Menge zurückgewinnen.

Im Rahmen dieser Erfindung werden bevorzugt lineare, wasserunlösliche Polysaccharide verwendet, weiche mit Hilfe von biotechnischen oder gentechnischen Methoden allgemeiner Definition gewonnen werden können. Eine besonders vorteilhafte Ausführungsform für die hier beschriebene Erfindung ist die Herstellung in einem biotechnischen Verfahren, insbesondere in einem biokatalytischen Verfahren.

Lineare Polysaccharide hergestellt durch Biokatalyse (ebenso: Biotransformation) im Rahmen dieser Erfindung bedeutet, daß das lineare Polysaccharid durch katalytische Reaktion von monomeren Grundbausteinen wie oligomeren Sacchariden, z. B. von Mono- und / oder Disacchariden hergestellt wird, indem ein sogenannter Biokatalysator, üblicherweise ein Enzym, unter geeigneten Bedingungen verwendet wird, Bevorzugt wird Poly(1,4-alpha-D-glukan) mittels Polysaccharidsynthasen und / oder Stärkesynthasen und /oder Glykosyltransfierasen und / oder alpha-1,4-Glukantransferasen und / oder Glycogensynthasen und / oder Amylosucresen und / oder Phosphorylasen hergestellt.

Denkbar sind ebenfalls lineare Polysaccharide aus Fermentation. Dies sind im Rahmen dieser Erfindung lineare Polysaccharide, die durch fermentative Prozesse unter der Verwendung in der Natur vorkommender Organismen, wie Pilze, Algen oder Mikroorganismen oder unter der Verwendung von in der Natur nicht vorkommender Organismen, die durch Modifizierung von natürlichen Organismen, wie Pilze, Algen oder Mikroorganismen, mittels gentechnischer Methoden allgemeiner Definition gewonnen werden können,

Darüber hinaus können lineare Polysaccharide zur Herstellung der in der vorliegenden Erfindung beschriebenen Retardtablette aus nicht-linearen Polysacchariden, die Verzweigungen enthalten, gewonnen werden, indem sie mit einem Enzym behandelt werden und unter Spaltung (z. B. mittels Enzyme, wie Amylase, iso-Amylase, Gluconohydrolase, Pullulanase u. a.) und Abtrennung der Verzweigungen lineare Polymere daraus erhalten werden können.

Die Molekulargewichte M_{w} der erfindungsgemäß verwendeten linearen Polysaccharide können in einem weiten Bereich von 10³ g/mol bis 10⁷ g/mol, variieren, vorzugsweise liegen die Molekulargewichte M_{w} im Bereich von 2 x 10³ g/mol bis 5 x 10⁴ g/mol, insbesondere 3 x 10³ g/mol bis 2 x 10⁴ g/mol. Für das vorzugsweise verwendete lineare Polysaccharid Poly(1,4-alpha-D-glukan) werden entsprechende Bereiche verwendet.

Die Molekulargewichtsverteilung bzw. Polydispersität M_{w}/Mₙ kann in weiten Bereichen je nach Herstellungsmethode des Polysaccharids variieren. Bevorzugt wird eine Polydispersität von 1,01 bis 50 eingesetzt, besonders bevorzugt von 1,5 bis 15. Dabei nimmt die Polydispersität mit einer bimodalen Verteilung der Molekulargewichte zu, wobei dies die Eigenschaften der Tablettenformulierung nicht negativ beeinflußt.

Mischungen von erfindungsgemäßen linearen Polysacchariden mit nicht-linearen Polysacchariden werden nicht ausgeschlossen. Unter einer "kontrollierten Wirkstoffabgabe" wird verstanden, daß der Wirkstoffe unter Akzeptanz einer den Umständen entsprechenden statistischen Abweichung nach einer bestimmten Zeit und / oder Zeitdauer in einer für den biologischen Organismus vorteilhaften Dosis freigesetzt wird.

Diese Definition beinhaltet auch Extreme, Zum einen die spontane Freigabe aller in der Formulierung vorliegenden Wirkstoffen innerhalb einer auf den Wert Null zusehenden Zeitdauer. Zum anderen die minimal erforderliche Menge/Dosis zur Erzielung eines therapeutischen Effeks über eine lange, gar unendliche Zeitdauer, mindestens einer Zeitdauer die notwendig ist, sämtliche in der Formulierung vorliegende Wirkstoffe freizusetzen.

Daher wird für die hier vorliegende Retardformulierung synonym von einer Depotformulierung oder Formulierung mit verzögerter Freisetzung gesprochen. Als "Wirkstoff wird jede biologisch aktive Substanz und Substanzkombination im weitesten Sinne angesehen (ausdrücklich im Human- und Veterinärbereich), insbesondere zur medizinischen Indikation. Insbesondere: Analgetika, Anginal Präparationen, Antiallergika, Antihistamine, Anti-inflammatories, Bronchodilatoren, Bronchospasmolytika, Diuretika, Anticholinergetika, Anti-Adhäsions Moleküle, Zytokin Modulatoren, biologisch aktive Endonucleasen, rekombinante Human-DNasen, Neurotransmitter, Leukotrin Inhibitoren, vasoaktive Intestinal Peptide, Endothelin Antagonisten, Analeptika, Analgetika, Lokalanästhetika, Narkosemittel, Antiepileptika, Antikonvulsiva, Antiparkinsonmittel, Antiemetika, das Hormonsystem regulierende oder stimulierende Verbindungen, das Herz-Kreislauf-System regulierende oder stimulierende Verbindungen, den Respirationstrakt System regulierende oder stimulierende Verbindungen, Vitamine, Spurenelemente, Antioxidantien, Zytostatika, Antimetabolite, Antiinfektiva, Immunmodulatoren, Immunsuppressiva; Antibiotika, Proteine, Peptide, Hormone, Wachstumshormone, Wachstumsfaktoren, Xanthine, Vakzine, Steroide, beta₂-Mimetika.

"Therapeutischer Effekt" im Sinne dieser Erfindung bedeutet, daß eine therapeutisch effektive Menge eines Wirkstoffs an den angestrebten Zielort gelangt, dort seine Wirkung entfaltet, und eine physiologische Reaktion bewirkt. Der palliative und / oder kurative Effekt wird einbezogen.

"Biokompatibel" im Sinne dieser Erfindung bedeutet, daß die eingesetzten Polysaccharide einem vollständigen biologischen Abbau unterzogen werden und keine Anreicherung im Organismus erfolgt. Unter biologischen Abbau wird dabei jedweder in vivo ablaufende Vorgang verstanden, der zu einem Abbau oder Zerstörung des Polymers führt. Insbesondere fallen ebenfalls hydrolytische oder enzymatische Prozesse in diesen Bereich. Für die Biokompatibilität der Polysaccharide sowie von dessen Abbauprodukten (Metabolite) ist nicht zuletzt auch der naturidentische Charakter der eingesetzten Polysaccharide von hoher Bedeutung. Daher sind die erfindungsgemäß verwendeten Polysaccharide für den therapeutischen, diagnostischen oder prophylaktischen Einsatz besonders geeignet. Der Begriff "pharmazeutisch akzeptabel" im Sinne dieser Erfindung bedeutet, daß ein Träger für einen Wirkstoff, ein Hilfsmittel oder auch sogenanntes Excipient, durch ein lebendes Wesen aufgenommen werden kann, ohne daß signifikante Nebenwirkungen für den Organismus entstehen.

Die Herstellung der Tabletten erfolgt durch Mischen der Ausgangskomponenten, wobei das lineare Polysaccharid mit dem Wirkstoff zusammen nach bekannten Methoden gemischt oder homogenisiert wird, z. B. mit Hilfe einer Kugelmühle, Der Wirkstoff kann eine Konzentration bis zu 50 % einnehmen, wobei bevorzugt eine Konzentration zwischen 1 und 20 %, besonders bevorzugt zwischen 5 und 15 % verwendet wird. Weitere übliche Hilfs- und Zusatzmittel können eingesetzt werden. Die Summe aus Wirkstoff und erfindungsgemäßem Polysaccharid an der Gesamtzusammensetzung (einschließlich etwaiger Hilfs- und Zusatzmittel) soll mindestens 50 % betragen, bevorzugt sind jedoch 70 bis 100 %, besonders bevorzugt sind 85 bis 98 %. Die Zusammensetzung der Hilfsstoffe kann in weiten Bereichen variieren, wobei die Verhältnisse der Zusammensetzung von den Wechselwirkungen zu dem Wirkstoff und dem linearen wasserunlöslichen Polysaccharid abhängen.

Als Hilfsmittel bei der Tablettenherstellung sowie des vorgelagerten Mischvorgangs können Lösungsmittel eingesetzt werden, wobei leichtflüchtige Lösungsmittel bevorzugt sind.

Der Grundkörper des erfindungsgemäßen Polysaccharids zur Tablettenherstellung kann ein Mikropartikel darstellen, wie es durch die Patentanmeldung (DPA, Az: 197 37 481.6) beschrieben ist, Der einfache Mischvorgang zur Herstellung der Rohmasse oder Rohmischung der Tablette wird bevorzugt angewendet. Diese Herstellungsweise der Tabletten können die Eigenschaften der Tablette beeinflussen. Es ist zum Beispiel möglich, den Wirkstoff durch Aufsprühtechniken, zum Beispiel im Wirbelschichtverfahren oder durch Coating in einer Suspension des erfindungsgemäß verwendeten Polysaccharids, direkt an oder auf dem Grundkörper des Polysaccharids zu koppeln, Dabei sind Saugprozesse einsetzbar, bei denen die poröse Gestalt der Mikropartikel ausgenutzt wird, um den Wirkstoff in einer Lösung aufzusaugen (Schwammcharakter), oder Sprühtrocknungstechniken. Hierbei wird eine Lösung, Suspension oder Emulsion eines linearen Polysaccharids und des Wirkstoffs mittels bekannter Sprühtechnologie getrocknet. Bei Lösungen werden entsprechende organische Lösungsmittel eingesetzt. Höhere Temperaturen oder Drucke, sowie überkritische Verfahren können dabei helfen, für kurze Zeiträume notwendige Löslichkeiten zu erzeugen.

Die während der Tablettenherstellung angewendeten Drucke können in weiten Bereichen variiert werden. Druckvariationen können gezielt dazu eingesetzt werden mit den erfindungsgemäß beschriebenen Polysacchariden einen zusätzlich sich positiv auswirkenden Retard-Effekt zu erzielen. Die Drucke können in weiten Bereichen variieren von 1 MPa bis 10³ MPa. (10⁵ Pa = 1 bar). Bevorzugt sind Drucke im Bereich von 10 MPa bis 300 MPa einzusetzen, besonders vorteilhaft Drucke im Bereich von 100 MPa bis 250 MPa.

Die nachfolgenden Beispiele und Figuren dienen zur näheren Erläuterungen der Erfindung, ohne dieselbe auf in den Beispielen beschriebenen Produkte und Ausführungsformen einzuschränken.

### Beispiele

Die folgenden Beispiele beziehen sich insbesondere auf die Herstellung der Mikropatikel, wie in der Patentanmeldung (DPA, AZ: 197 37 481.6) beschrieben, auf diese wird ausdrücklich Bezug genommen. Des weiteren wird eine besonders vorteilhafte Methode zur Herstellung von Poty(1,4-atpha-D-gtukan) in WO 95/31553 beschrieben.

### Beispiel 1

### In-vitro-Produktion von Poly(1,4-α-D-glukan) in einem biokatalytischen Prozeß mit Hilfe des Enzyms Amylosucrase

In einem sterilisierten (Dampfsterilisation) 15 I Gefäß werden 10 I einer 20 %igen Saccharose Lösung gegeben. Der mittels einer Fermentation erhaltene Amylosucrase enthaltende Enzymextrakt wird in einer Portion zu der Saccharoselösung gegeben. Die Enzymaktivität beträgt 16 units (1 unit enspricht der Umsetzung von 1 µmol Saccharose pro Minute pro mg Enzym). Die Apparatur wird mit einem ebenfalls sterilisierten KPG-Rührer versehen. Das Gefäß wird verschlossen und bei 40 °C aufbewahrt und gerührt. Nach einiger Zeit bildet sich ein weißer Niederschlag. Die Reaktion wird nach einer Zeitdauer von 180 Stunden beendet. Der Niederschlag wird abfiltriert und zur Abtrennung niedermolekularer Zucker mehrfach gewaschen. Der im Filter verbleibende Rückstand wird bei Temperaturen zwischen 30 und 40 °C im Trockenschrank unter Anlegung eines Vakuums mit Hilfe einer Membranpumpe (Firma Vacuubrand GmbH & Co, CVC 2) getrocknet. Die Masse beträgt 685 g (Ausbeute 69 %).

### Beispiel 2

### Charakterisierung des mit Amylosucrase synthetisierten Poly(1,4-α-D-glukan) aus Beispiel 1 mittels Gelpermeationschromatographie

Es werden 2 mg des Poly(1,4-(α-D-glukan) aus Beispiel 1 bei Raumtemperatur in Dimethylsulfoxid (DMSO, p. a. von Riedel-de-Haen) gelöst und filtriert (2 mm Filter). Ein Teil der Lösung wird in eine Gelpermeationschromatographie Säule injeziert. Als Elutionsmittel wird DMSO verwendet. Die Signalintensität wird mittels eines Rl-Detektors gemessen und gegen Pullulanstandards (Firma Polymer Standard Systems) ausgewertet. Die Flußrate beträgt 1.0 ml pro Minute.

Die Messung ergibt ein Zahlenmittel des Molekulargewichts (Mₙ) von 2.700 g/mol und ein Gewichtsmittel des Molekulargewichts (M_{w}) von 11.700 g/mol. Dies entspricht einer Dispersität von 4,3.

### Beispiel 3

### Herstellung von Mikropartikeln aus Poly(1,4-α-D-glukan)

400 g Poly(1,4-α-D-giukan) werden in 2 l Dimethylsulfoxid (DMSO, p. a. von Riedel-de-Haen) bei 60 °C innerhalb von 1,5 h gelöst. Dann wird eine Stunde bei Raumtemperatur gerührt. Die Lösung wird in 20 l bidestilliertem Wasser unter Rühren durch einen Tropftrichter über einen Zeitraum von 2 h hinzugegeben. Der Ansatz wird für 40 h bei 6 °C gelagert. Es bildet sich eine feine Suspension aus. Der Partikel werden abgetrennt, indem zunächst der Überstand abdekantiert wird. Der Bodensatz wird aufgeschlämmt und in kleinen Portionen zentrifugiert (Ultrazentrifuge RC5C: je 5 Minuten bei 5000 Umdrehungen pro Minute). Der feste Rückstand wird insgesamt drei Mal mit bidestilliertem Wasser aufgeschlämmt und erneut zentrifugiert. Die Feststoffe werden gesammelt und die Suspension von ca. 1000 ml gefriergetrocknet (Christ Delta 1-24 KD). Es werden 283 g weißer Feststoff isoliert (Beispiel 3a: Ausbeute 71 %). Die gesammelten Überstände werden bei einer Temperatur von 18 °C über Nacht verwahrt. Die Aufarbeitung erfolgt wie beschrieben. Es werden weitere 55 g des weißen Feststoffs isoliert (Beispiel 3b: Ausbeute 14 %). Die Gesamtausbeute beträgt 85 %.

### Beispiel 4

### Entschwefelung der Mikropartikel aus Beispiel 3

Zur Abtrennung in den Partikeln verbliebenen Dimethylsulfoxids wird wie folgt vorgegangen 100 g der Amylosepartikel aus Beispiel 9 werden in 1000 ml entionisiertem Wasser gegeben. Der Ansatz wird für 24 h unter leichtem Schwenken sich selbst überlassen. Die Abtrennung der Partikel erfolgt wie in Beispiel 9 beschrieben (Ultrazentrifuge RC5C: je 15 Minuten, 3000 U / min. Nach der Gefriertrocknung ergibt sich eine Auswaage von 98,3 g (98 % Ausbeute). Die Schwefelbestimmung durch Elementaranalyse ergibt folgende Werte (Prüfmethode Verbrennung und lR-Detektion):

| | |
|---|---|
| Schwefelgehalt der Partikel aus Beispiel 2: | 6 % +/- 0,1 % |
| Schwefelgehalt der Partikel aus Beispiel 3: | < 0,01 % |

### Beispiel 5

### Untersuchungen der Mikropartikel aus dem Beispiel 3 mittels Elektronenmikroskopie

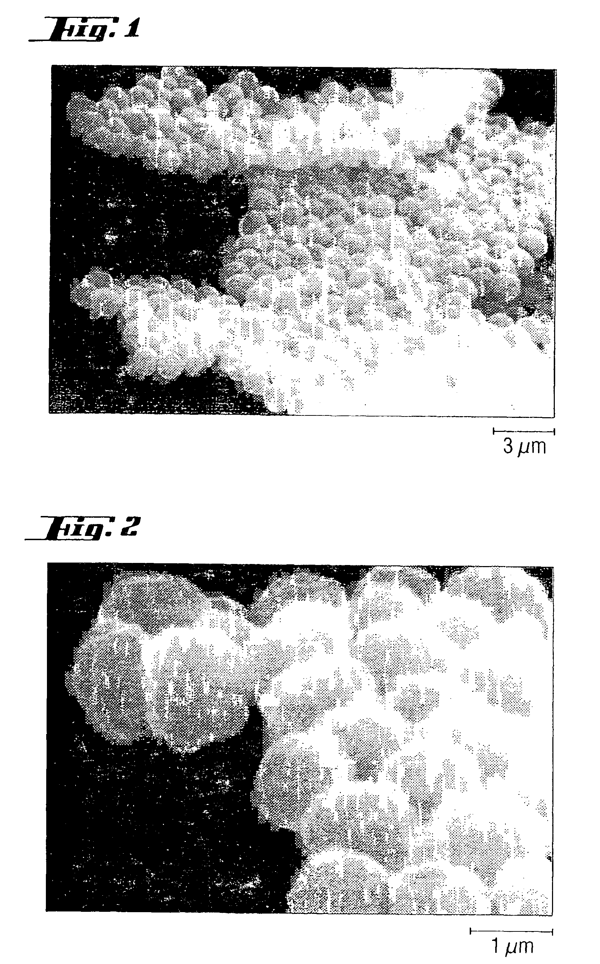

Zur Charakterisierung der Partikel werden Rasterelektronenmikroskopaufnahmen (REM) (Camscan S-4) durchgeführt. Die Figuren 1 und 2 zeigen Aufnahmen der Partikel, die verdeutlichen, daß es sich um sphärische, sehr einheitliche Partikel hinsichtlich der Form, Größe und Oberflächenrauigkeit handelt.

### Beispiel 6

### Untersuchungen der Größenverteilungen der Partikel aus Beispiel 3

Zur Charakterisierung der Größenverteilungen der Partikel aus den Beispielen 1 und 9 wurden Untersuchungen mit einem Mastersizer durchgeführt (Fa. Malvern Instruments). Die Untersuchung erfolgte im Fraunhofer Modus (Auswertung: multimodal, Anzahl) mit einer Dichte von 1,080 g/cm³ und Volumenkonzentration im Bereich von 0.012 % bis 0,014 %.

**Tabelle 1:**

| Charakterisierung der Partikeldurchmesser der Mikropartikel aus Beispiel 3. | | | | | | |
|---|---|---|---|---|---|---|
| **Beispiel** | **Durchmesser** | | | **Partikelverteilung** | | |
| **No.** | **Dn*¹ (mm)** | **Dw*² (mm)** | **dw/ dn*³** | **d (10 %)*⁴ (mm)** | **d (50 %)*⁵ (mm)** | **d (90 %)*⁶ (mm)** |
| 3a | 1,664 | 4,184 | 2,541 | 0,873 | 1,504 | 2,624 |
| 3b | 0,945 | 2,345 | 2; 481 | 0,587 | 0,871 | 1,399 |

| | | | | | | |
|---|---|---|---|---|---|---|
| *¹ dn: Zahlenmittelwert des Durchmessers *² dw: Gewichtsmittelwert des Durchmessers *³ dw/dn: Dispersität der Partikeldurchmesser ^{*4} d(10 %): 10 % aller Partikel haben einen kleineren Durchmesser als der angegebene Wert *⁵ d(50 %): 50 % aller Partikel haben einen kleineren Durchmesser als der angegebene Wert *⁶ d(90 %): 90 % aller Partikel haben einen kleineren Durchmesser als der angegebene Wert | | | | | | |

### Beispiel 7

### Allgemeines Herstellungsverfahren von Tabletten aus Mikropartikeln enthaltend Poly(1,4-α-D-glukan)

Es werden 270 mg Tablettenhilfsstoff (Poly(1,4-α-D-gluka)) und 30 mg Wirkstoff werden in einer Kugelmühle (Firma: Retsch MM2000) 10 Minuten bei einer Amplitude von 100 (Herstellerangabe) gemahlen. Von der homogenisierten Menge werden 250 mg entnommen und in ein Preßwerkzeug (Perkin Elmer; Durchmesser des Stempels 13 mm) überführt. Das Preßwerkzeug wird unter eine Presse gestellt (Firma: Perkin Elmer, Hydraulische Presse). Anschließend wird die Masse bei einem Druck von 2 t für 10 Minuten gepreßt. Nach Entspannung der Apparatur wird die fertige Tablette vorsichtig entnommen und für die weitere Charakterisierung, z. B. Stabilitätsmessungen oder Freisetzungsversuche, aufbewahrt.

Im folgenden werden für Vergleichszwecke Tabletten aus bekannten Tablettenformulierungsmaterialien (Vergleichsbeispiele) bereitgestellt, wie: mikrokristalline Cellulose (Avicel^{™}), Kartoffelstärke (Toffena^{™}-Südstärke), sowie Polyacrylate (Eudragite^{™}-Rhöm).

### Beispiel 8

### Bestimmung der Wirkstoff-Freisetzung in Abhängigkeit von der Zeit

Die Freisetzung der gemäß Beispiel 7 hergestellten Tabletten wird wie folgt bestimmt. Eine Tablette wird in 25 ml Wasser (entionisiertes Wasser) in einen 50 ml-Erlenmeyerkolben gegeben. Die Öffnung wird mit Parafilm abgedeckt. Der Kolben wird auf einem Schüttler (Firma: IKA Labortechnik; KS 125 basic) befestigt. Der Schüttler wird bei einer Einstellung von ca. 150 pro Minute betrieben. Nach bestimmten Zeiten werden Proben - ca. 1,5 ml - aus dem Überstand der entstandenen Lösung entnommen. Von diesem Volumen wird eine ausreichende Menge in eine Einwegküvette (Firma: Sarstedt No. 67.741) überführt und am Spektrometer (Firma: Kontrom Instruments, Uvikon 860) vermessen. Es gelten die für die einzelnen Wirkstoffe oder Modellsubstanzen auftretenden Absorptionsmaxima.

### Beispiel 9

### Absorptionsmaxima weiterer Wirkstoffe

Die Absorptionsmaxima weiterer Wirkstoffe wurden wie in Beispiel 10 bestimmt. Alle genannten Wirkstoffe führen zu vergleichbaren Ergebnissen bei der Beobachtung eines Retardeffekts, wodurch Rückschlüsse auf die Vielfalt möglicher Anwendungen geschlossen werden können.

**Tabelle 2:**

| Absorptionsmaxima verschiedener untersuchter Wirkstoffe | |
|---|---|
| Wirkstoff | Absorptionsmaximum |
| Vitamin B12 | 549 nm^{*2} |
| Theophyllin | 271 nm^{*3} |
| Ramorelix^{™} | 276 nm^{*3} |
| Coffein | 272 nm^{*3} |
| Iloperidon | 274 nm^{*3} |
| Buserelin^{*1} | 278 nm^{*3} |
| Minocyclinhydrochlorid | 278 nm^{*3} |
| Tetracyclinhydrat | 269 nm^{*3} |
| Phenylephrin | 272 nm^{*3} |

| | |
|---|---|
| ^{*1} Struktur:5-Oxo-L-prolyl-L-histidy-L-tryptophyl-L-seryl-L-tyrosyl-O-tert-butyl-D-seryl-L-leucyl-L-arginyl-N-ethyl-L-prolinamid ^{*2} Einwegküvette (Firma: Sarstedt No. 67.741) ^{*3} Quarzküvette (Firma: Hellma, Suprasil^{®}) | |

### Beispiel 10

### Aufnahme einer Eichkurve für einen Wirkstoff am Beispiel der Modellverbindung Vitamin B₁₂

Eine 1 %ige Stammlösung wird durch Einwaage von 100 mg Vitamin B₁₂ in einen 10 mL Meßkolben und Auffüllung mit E-Wasser bis zur Eichmarke hergestellt. Daraus werden durch eine Verdünnungsreihe Konzentrationen von 0,005 %, 0,01 % und 0,02 % hergestellt und im Spektrometer (Firma: Kontrom Instruments, Uvikon 860) gemessen. Die Extinktionen werden beim Absorptionsmaximum von λ = 549 nm abgelesen. Weitere Meßpunkte sind nur dann notwendig, wenn Abweichungen von einer Geraden erkennbar sind. Diese Eichgerade dient als Ausgangspunkt für die Bestimmung der Konzentration im Überstand des Wirkstoff-Freisetzungsversuchs. Bei der Aufnahme von Eichgeraden anderer Wirkstoffe und Modellsubstanzen wird in analoger Weise bei der Datenerhebung verfahren.

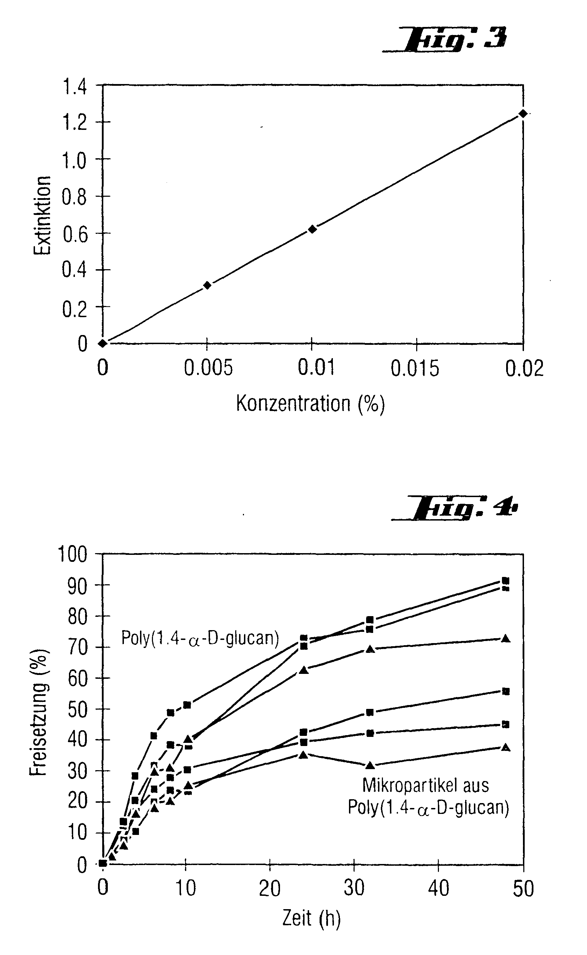

Die graphische Darstellung der Eichung für Vitamin B₁₂ (Extinktion in Wasser in Abhängigkeit von der Konzentration) ist in Figur 3 gezeigt.

### Beispiel 11

### Versuche zur Freisetzung von Vitamin B₁₂ aus Tabletten hergestellt mit Poly(1,4-α-D-glukan), sowie Mikropartikeln daraus

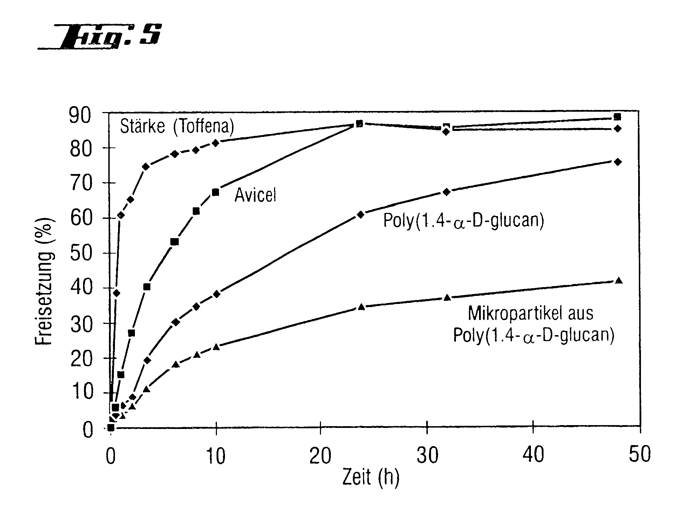

Die Extinktionen des Überstands der in vitro Freisetzungsversuche werden, wie in Beispiel 10 beschrieben, nach bestimmten Zeiten gemessen. Um über die gemessene Extinktion aus der Eichkurve die korrespondierenden Konzentrationswerte zu erzielen, kann es notwendig sein, die Überstände im Verhältnis 1 : 1 0 zu verdünnen. Dieser Faktor findet dementsprechend Berücksichtigung.
In Tabelle 3 sind die Werte zusammenfassend dargestellt. In der korrespondierenden Figur 4 sind die Werte in der Graphik gegenübergestellt. Die maximal mögliche Konzentration - gemäß Beispiel 8 ist dies 0,1 % - wurde zur übersichtlicheren Darstellung mit dem Wert einhundert Prozent gleichgesetzt, so daß dadurch auch eher eine Abschätzung möglich ist, welcher Grad der Vollständigkeit nach welcher Zeit bereits erreicht ist.

**Tabelle 3:**

| Konzentrationswerte des wäßrigen Überstands in Abhängigkeit von der Zeit für die erfindungsgemäß beschriebenen Tablettenhilfsmaterialien Poly(1,4-α-D-glukan) und Mikropartikel daraus. | | | | | | | | | | | |
|---|---|---|---|---|---|---|---|---|---|---|---|
| | Freisetzung (Stunden) | | | | | | | | | | |
| Tablettenhilfsmittel | 0 | 0,5 | 1 | 2 | 3,5 | 6 | 8 | 10 | 24 | 32 | 48 |
| poly(1,4-α-D-glukan) | 0 | 5,44 | 6,74 | 11,45 | 24,45 | 35,26 | 41,14 | 43,28 | 60,90 | 63,33 | 75,27 |
| Poly(1,4-α-D-glukan) | 0 | 2,23 | 4,87 | 7,57 | 15,32 | 26,11 | 27,20 | 34,78 | 55,02 | 61,21 | 54,30 |
| Poly(1,4-α-D-glukan) | 0 | 4,91 | 5,94 | 8,18 | 19,57 | 30,36 | 36,38 | 36,38 | 67,08 | 74,96 | 87,17 |
| Mikropartikel aus Poly(1,4-α-D-glukan) | 0 | 3,97 | 4,03 | 6,52 | 14,22 | 20,37 | 23,71 | 26,12 | 33,23 | 35,70 | 38,18 |
| Mikropartikel aus Poly(1,4-α-D-glukan) | 0 | 3.52 | 3,74 | 5,72 | 9,43 | 17,23 | 17,77 | 21,90 | 31,07 | 28,13 | 33,38 |
| Mikropartikel aus Poly(1,4-α-D-glukan) | 0 | 3,14 | 3,89 | 5,63 | 11,19 | 18,92 | 22,94 | 22,94 | 39,92 | 46,83 | 53,17 |

Die Freisetzung von Vitamin B₁₂ aus Tabletten verschiedener Hilfsstoffe: a) Poly(1,4-α-D-glukan) und b) Mikropartikel aus Poly(1,4-α-D-glukan) gezeigt.

### Beispiel 12

### Versuche zur Freisetzung von Vitamin B₁₂ aus Tabletten hergestellt mikrokristalliner Cellulose (Avicel^{™}) und Kartoffelstärke (Toffena^{™}) (Vergleichsbeispiele)

Die in Tabelle 4 dargestellten Ergebnisse wurden wie in Beispiel 11 beschrieben gemessen und errechnet. Figur 8 stellt vergleichend die Ergebnisse der erfindungsgemäß beschriebenen Tablettenhilfsstoffe Poly(1,4-α-D-glukan) und Mikropartikeln aus Poly(1,4-α-D-glukan) zu den Vergleichssubstanzen mikrokristalline Cellulose (Avicel^{™}) und Kartoffelstärke (Toffena^{™}) dar. Der Retardeffekt ist hier deutlich zu erkennen.

**Tabelle 4:**

| Konzentrationswerte des wäßrigen Überstands in Abhängigkeit von der Zeit für die Vergleichssubstanzen mikrokristalline Cellulose (Avicel^{™}) und Kartoffelstärke (Toffena^{™}) | | | | | | | | | | | |
|---|---|---|---|---|---|---|---|---|---|---|---|
| | Freisetzung (Stunden) | | | | | | | | | | |
| Tablettenhilfsstoff | 0 | 0,5 | 1 | 2 | 3,5 | 6 | 8 | 10 | 24 | 32 | 48 |
| mikrokristalline Cellulose (Avicel^{™}) | 0 | 6,99 | 13,38 | 34,31 | 52,40 | 65,53 | 75,12 | 77,13 | 78,36 | 72,80 | 79,75 |
| mikrokristalline Cellulose (Avicel^{™}) | 0 | 6,51 | 14,84 | 22,30 | 31,53 | 43,89 | 52,40 | 67,23 | 84,70 | 81,92 | 84,39 |
| mikrokristalline Cellulose (Avicel^{™}) | 0 | 4.81 | 17,68 | 26,12 | 37,51 | 50,54 | 57,50 | 57,50 | 95,05 | 100 | 100 |
| Kartoffelstärke (Toffena^{™}) | 0 | 7,99 | 74,65 | 77,28 | 76,51 | 78,98 | 78,98 | 78,98 | 83,15 | 80,53 | 84,08 |
| Kartoffelstärke (Toffena^{™}) | 0 | 12,18 | 33,54 | 39,57 | 65,07 | 69,86 | 77,43 | 84,08 | 87,48 | 85,32 | 82,07 |
| Kartoffelstärke (Toffena^{™}) | 0 | 93,97 | 74,34 | 77,28 | 81,14 | 184,70 | 80,53 | 80,53 | 87,79 | 87,48 | 87,48 |

In Figur 5 ist die Freisetzung von Vitamin B₁₂ aus Tabletten verschiedener Hilfsstoffe gezeigt: a) Poly(1,4-α-D-glukan), b) Mikropartikel aus Poly(1,4-α-D-glukan), c) mikrokristalliner Cellulose (Avicel^{™}) (Vergleichsbeispiel) und d) Kartoffelstärke (Toffena^{™}) - Vergleichsbeispiel (Zur übersichtlicheren Darstellung wurden die Mittelwerte der in den Tabellen 3 und 4 angegebenen Werte berechnet).

### Beispiel 13

### Versuche zur Freisetzung von Theophyllin-Tabletten bestehend aus Poly(1,4-α-D-glukan) und Mikropartikeln aus Poly(1,4-α-D-glukan)

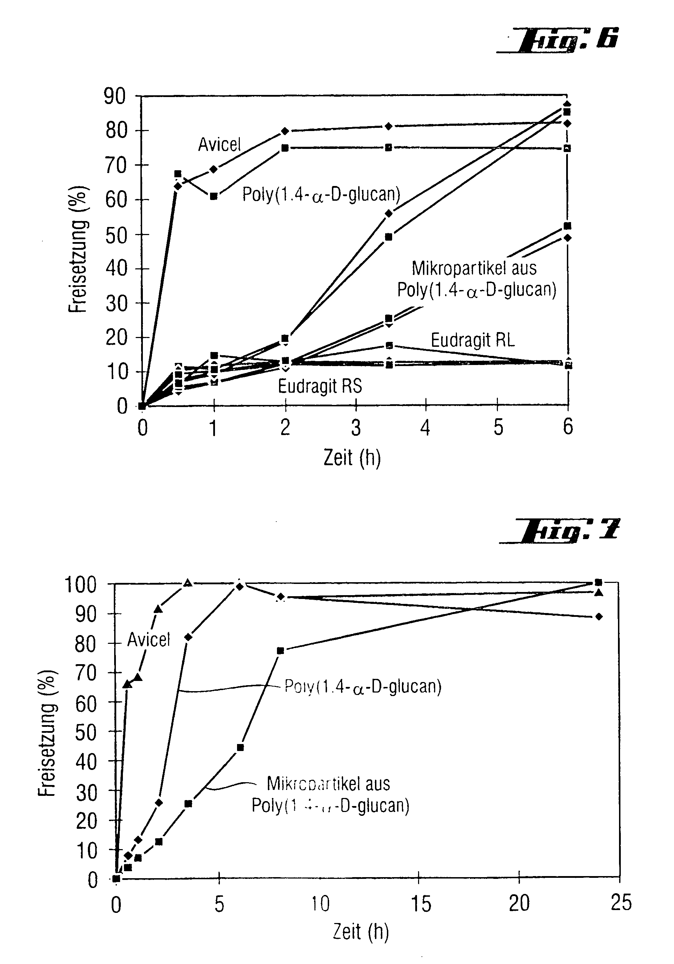

Die Freisetzungsversuche von Theophyllin aus Tabletten bestehend aus verschiedenen Tablettenhilfsstoffen werden analog zu Beispiel 12 durchgeführt. Als Tablettenhilfsstoff wird Poly(1,4-α-D-glukan) verwendet, welches direkt aus der Biokatalyse nach entsprechenden Aufarbeitungsprozessen (vgl. Beispiel 1) gewonnen wurde, sowie Mikropartikel aus Poly(1,4-α-D-glukan). Die Ergebnisse sind in Figur 6 dargestellt. Es wurden je eine Doppelbestimmung durchgeführt, die erneut die hohe Reproduzierbarkeit der Ergebnisse unter Beweis stellt.

### Beispiel 14

### Versuche zur Freisetzung von Theophyllin-Tabletten bestehend aus c) mikrokristalliner Cellulose (Avicel^{™}) d) Eudragit RS^{™} und e) Eudragit RL^{™} (Vergleichsbeispiele)

Die Freisetzungsversuche von Theophyllin aus Tabletten bestehend aus den Vergleichssubstanzen Tablettenhilfsstoffen wird analog zu Beispiel 14 durchgeführt. Als Tablettenhilfsstoff werden mikrokristalliner Cellulose (Avicel^{™}), Eudragit RS^{™} und Eudragit RL^{™} eingesetzt. Die Ergebnisse sind in Figur 6 dargestellt. Es wurden je eine Doppelbestimmung durchgeführt.

In Figur 6 sind die Freisetzungsprofile aus Beispiele 13 vergleichend gegenübergestellt.

Die Figur 6 zeigt die Freisetzung von Theophyllin aus Tabletten verschiedener Hilfsstoffe: a) Poly(1,4-α-D-glukan), b) Mikropartikel aus Poly(1,4-α-D-glukan), c) mikrokristalliner Cellulose (Avicel^{™}), d) Eudragit RS^{™} und e) Eudragit RL^{™}.

### Beispiel 15

### Versuche zur Freisetzung von Theophyllin-Tabletten bestehend aus Poly(1,4-α-D-glukan), sowie Mikropartikeln aus Poly(1,4-α-D-glukan) und mikrokristalliner Cellulose (Avicel^{™}) (Vergleichsbeispiel) in künstlichem Magensaft

Die Freisetzungsversuche von Theophyllin aus Tabletten mit den Tablettenhilfsstoffen Poly(1,4-α-D-glukan) (inkl. Mikropartikel) und mikrokristalliner Cellulose (Avicel^{™}) in künstlichem Magensaft wurde analog zu Beispiel 8 durchgeführt (künstlichem Magensaft: 2 g Natriumchlorid, 3,2 g Pepsin, 7 ml konzentrierte Salzsäure (HCl_{aq}), aufzufüllen auf ein Liter Gesamtvolumen mit entionisiertem Wasser). Auch bei der Verwendung eines Medium, welches die natürliche Umgebung widerspiegelt sind in reproduzierbarer Weise die Retardeffekte in der Freisetzung zu beobachten.

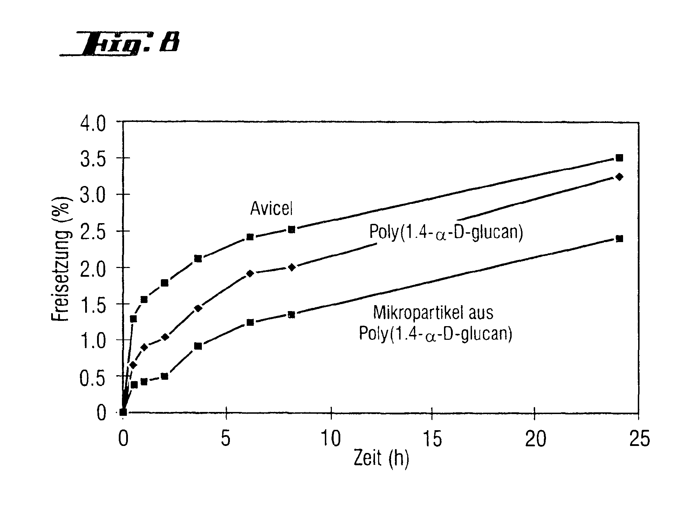

Die Figur 7 zeigt die Freisetzung von Theophyllin aus Tabletten verschiedener Hilfsstoffe: a) Poly(1,4-α-D-glukan), b) Mikropartikel aus Poly(1,4-α-D-glukan) und c) mikrokristalliner Cellulose (Avicel^{™}) (Vergleichsbeispiel).

### Beispiel 16

### Versuche zur Freisetzung von Ramorelix^{™} Tabletten bestehend aus Poly(1,4-α-D-glukan) und daraus hergestellten Mikropartikeln

Die Freisetzungsversuche von Ramorelix^{™} wurde entsprechend der bisher geschilderten Beispiele durchgeführt. Bei Ramorelix^{™} handelt es sich um einen LHRH-Antagonisten mit folgender Aminosäuresequenz (Struktur): 1 -(N-Acetyl-3-(2-naphthyl)-D-alanyl-p-chloro-D-phenylalanyl-D-tryptophyl-L-seryl-L-tyrosyl-0-(6-deoxy-alpha-L-mannopyranosyl)-D-seryl-L-leucyl-L-arginyl-L-prolyl)semicarbazidacetat. Als Freisetzungsmedium wurde jedoch künstlicher Magensaft anstelle von entionisiertem Wasser (E-Wasser) eingesetzt. Die Rezeptur für künstlichen Magensaft ist: 2 g Natriumchlorid, 3,2 g Pepsin, 7 ml konzentrierte Salzsäure (HCl_{aq}) auf ein Liter Gesamtvolumen mit entionisiertem Wasser aufgefüllt. Der pH-Wert der Lösung beträgt 1,2.

### Beispiel 17

### Versuch zur Freisetzung von Ramorelix^{™} Tabletten bestehend aus mikrokristalliner Cellulose (Avicel^{™}) (Vergleichsbeispiel)

Die Freisetzungsversuche werden, wie in Beispiel 8 angegeben, durchgeführt. Als Freisetzungsmedium dient künstlicher Magensaft.

Die Figur 8 zeigt die Freisetzung von Ramorelix^{™} aus Tabletten verschiedener Hilfsstoffe: a) Poly(1,4-α-D-glukan), b) Mikropartikel aus Poly(1,4-α-D-glukan) und c) mikrokristalliner Cellulose (Avicel^{™}) (Vergleichsbeispiel).

### Beispiel 18

### Bestimmung der Löslichkeit von Polysacchariden

Es werden 100 mg Poly(1,4-α-D-glukan) in 5 ml bidestilliertem Wasser gegeben. Das Reaktionsgefäß wird unter Rühren (Magnetrührer) langsam aufgehetzt. Es wird in einem Stufenprogramm mit Abständen von zwanzig Grad erhitzt und mit dem Auge beobachtet. Bei Temperaturen von 40 °C, 60 °C, 80 °C und 100 °C sind keine Veränderungen zu beobachten. Gemäß dieser Beobachtungen ist die Verbindung die Eigenschaft "wasserunlöslich" zuzuordnen.

### Beispiel 19

### Bestimmung der Löslichkeit von Polysacchariden und Klassifizierung nach Deutschem Arzneimittelbuch (DAB)

564 mg Poly(1,4-α-D-glukan) werden in ca. 0,5 L bidestilliertem Wasser bei 1,3 bar und 130°C für 1,5 Stunden in einem Autoklaven erhitzt (Apparat Certoclav). Von dem Reaktionsgefäß ist zuvor das Gewicht gemessen worden. Danach wird die Apparatur entspannt und bei Raumtemperatur abgekühlt. Der Inhalt wird gewogen. Er enspricht 501,74 g. Nach weiteren 24 Stunden wird zentrifugiert und dekantiert. Der feste Rückstand wird getrocknet und ausgewogen: 468 mg. Daraus errechnet sich ein gelöster Anteil von 96 mg. Bezogen auf das eingesetzte Lösungsmittel errechnet sich daraus, daß für 1 mg Poly(1,4-α-D-glukan) 5226 mg Wasser notwendig sind. Gemäß der Klassifizierung nach Deutschem Arzneimittelbuch ergibt sich daraus die Einteilung, daß diese Substanz "sehr schwer löslich" ist, da zwischen 1.000 und 10.000 Teilen Lösungsmittel notwendig sind, um 1 Teil der Substanz in Lösung zu bringen. Dies ist von den 7 Klassen zur Einteilung der Löslichkeit (von "sehr leicht löslich" (Klasse 1) bis "praktisch unlöslich"(Klasse 7) die Klasse Nummer 6.

### Beispiel 20

### Bestimmung der Löslichkeit von Polysacchariden und Klassifizierung nach Deutschem Arzneimittelbuch (DAB)

Der Versuch wird wie in Beispiel 19 durchgeführt. Der einzige Unterschied bildet ein Kühlprozeß, der nach der Autoklavbehandlung und dem Abkühlen auf Raumtemperatur nachgeschaltet wird. Das Substanzgemisch wird für 3 Stunden bei 5 °C aufbewahrt.

Es werden 526 mg Poly(1,4-α-D-glukan) auf ca. 480 mL bidestilliertem Wasser eingewogen. Nach der thermischen Behandlung ergibt sich eine Auswaage von 468,09 g. Das getrocknete Sediment beträgt 488 mg. Demnach sind 38 mg des Poly(1,4-α-D-glukans) in Lösung gegangen. Dies entspricht einem Verhältnis von 1 mg Substanz zu 12.318 Teilen Lösungsmittel. Demnach ist die Substanz nach dieser Behandlungsmethode in Klasse Nummer 7 nach DAB einzustufen und danach als praktisch unlöslich zu klassifizieren, weil mehr als 10.000 Teile Lösungsmittel für ein Teil Substanz benötigt werden.

## Patentansprüche

1. Retardtablette, enthaltend mindestens ein wasserunlösliches, lineares Poly(1,4-alpha-D-Glucan) und einen oder mehrere Wirkstoffe, wobei das Poly(1,4-alpha-D-Glucan) ganz oder teilweise in Form von Mikropartikeln vorliegt.

2. Retardtablette nach Anspruch 1, enthaltend mindestens ein wasserunlösliches lineares Poly(1,4-alpha-D-Glucan), welches in einem biotechnischen Verfahren hergestellt wurde.

3. Retardtablette nach Anspruch 1, enthaltend mindestens ein wasserunlösliches lineares Poly(1,4-alpha-D-Glucan), welches in einem biokatalytischen Prozess hergestellt wurde.

4. Retardtablette nach Anspruch 1, enthaltend mindestens ein wasserunlösliches lineares Poly(1,4-alpha-D-Glucan), welches in einem fermentativen Prozess hergestellt wurde,

5. Retardtablette nach einem oder mehreren der Ansprüche 1 bis 4, **dadurch gekennzeichnet, dass** diese biokompatibel und/oder pharmazeutisch akzeptabel ist.

6. Retardtablette nach einem oder mehreren der Ansprüche 1 bis 5, enthaltend weitere Zusatz- und Hilfsstoffe.

7. Verwendung der Retardtablette nach einem oder mehreren der Ansprüche 1 bis 6 zur Herstellung eines Medikaments zur kontrollierten Wirkstoffabgabe.

## Claims

1. A sustained-release tablet, containing at least one water-insoluble linear poly(1,4-alpha-D-glucan) and one or more active ingredients, wherein the poly(1,4-alpha-D-glucan) is entirely or partially present in the form of microparticles.

2. The sustained-release tablet according to claim 1, containing at least one water-insoluble linear poly(1,4-alpha-D-glucan), which has been prepared in a biotechnological process.

3. The sustained-release tablet according to claim 1, containing at least one water-insoluble linear poly(1,4-alpha-D-glucan), which has been prepared in a biocatalytic process.

4. The sustained-release tablet according to claim 1, containing at least one water-insoluble linear poly(1,4-alpha-D-glucan), which has been prepared in a fermentative process.

5. The sustained-release tablet according to one or more of claims 1 to 4, **characterized in that** it is biocompatible and/or pharmaceutically acceptable.

6. The sustained-release tablet according to one or more of claims 1 to 5, containing further additives and auxiliaries.

7. Use of the sustained-release tablet according to one or more of claims 1 to 6 for preparing a medicament for the controlled release of active ingredient.

## Revendications

1. Tablette retard, contenant au moins un poly (1,4-alpha-D-glucan) linéaire, insoluble dans l'eau et l'une ou plusieurs substances actives, où le poly (1,4-alfa-D-glucan), existe totalement ou partiellement sous forme de microparticules.

2. Tablette retard selon la revendication 1, contenant au moins un poly (1,4-alfa-D-glucan), linéaire, insoluble dans l'eau, qui a été obtenu par un procédé biotechnologique.

3. Tablette retard selon la revendication 1, contenant au moins un poly (1,4-alfa-D-glucan), linéaire, insoluble dans l'eau, qui a été obtenu par un procédé biocatalytique.

4. Tablette retard selon la revendication 1, contenant au moins un poly (1,4-alfa-D-glucan), linéaire, insoluble dans l'eau, qui a été obtenu par un procédé fermentatif.

5. Tablette retard selon l'une ou plusieurs des revendications 1 à 4, **caractérisée en ce que** celle-ci est biocompatible et/ou acceptable du point de vue pharmaceutique.

6. Tablette retard selon l'une ou plusieurs des revendications 1 à 5, contenant d'autres substances supplémentaires et auxiliaires.

7. Utilisation tablette retard selon l'une ou plusieurs des revendications 1 à 6, pour l'obtention d'un médicament pour la libération contrôlée de substance active.
